# EUROPEAN PATENT APPLICATION

(11) **EP 2 087 834 A1**
(43) Date of publication of application: **12.08.2009**
(21) Application number: 09152413.2
(22) Date of filing: 09.02.2009
(51) Int. Cl.: A61B 1/31, A61B 17/28

(54) **Surgical instrument**

(30) Priority: 07.02.2008 US 63938 P
(71) Applicant: Karl Storz GmbH & Co. KG, 78532 Tuttlingen (DE)
(72) Inventor: Regadas, F. Sergio P., 60810-180, Fortaleza-Ceara (BR)
(74) Representative: Witte, Weller & Partner

(57) **Abstract**

A surgical instrument (100) adapted for use during transanal endoscopy is disclosed. The surgical instrument has a shaft (104) having a proximal end (120) and distal end (110). A working part (170), for example a scissors or gripper, is attached to the distal end of the shaft, and a handle portion (140) is attached to proximal end of the shaft. The handle portion is actuatable to actuate said working part via a force transmission member (106). The shaft further includes a linear insertion portion (130) having a longitudinal axis. The proximal end of the shaft is laterally displaced relative to the longitudinal axis of the insertion portion. This configuration increases the ability to manipulate and actuate the surgical instrument during a transanal endoscopic procedure or similar surgical procedure.

## Description

### CROSS-REFERENCE TO RELATED APPLICATIONS

The present application claims the benefit under 35 U.S.C. § 119(e) of the Provisional Patent Application Serial No. 61/063,938 entitled "Surgical Devices for use in the Transanal Endoscopic Surgical Techniques" filed on February 7, 2008.

This Application is related to U.S. Patent Application entitled "Operating Anoscope for Transanal Endoscopic Microsurgery," filed on January 26, 2009 and having application number 12/359,697. The application is incorporated herein by reference.

### FIELD OF THE INVENTION

The present invention relates a surgical instrument. More particularly the present invention relates to a surgical instrument for use during surgical endoscopy. Even more particularly, the present invention relates to a surgical instrument for use during a transanal endoscopic surgery.

### BACKGROUND OF THE INVENTION

The technique of transanal endoscopic microsurgery (TEM) has been made available for clinical use since 1983. This technique is currently the only one-port system in endoscopic surgery by which there is a direct endoluminal approach to the target organ by using a natural opening of the body. The technique is useful in removing cancerous or other abnormal cells located in the rectal area or colon, which may cause rectal or colon cancer.

TEM involves a surgeon using a rectoscope to access the transanal cavity of a person or an animal. The surgeon is thus able to access the affected region through the use of the rectoscope. Typically, a rectoscope is short (200 mm long), straight, rigid, hollow metal tube, and typically has a small light bulb mounted at the end.

During TEM, a surgeon uses tools configured for the rectoscope to access the surgical site. This allows the surgeon to access the affected region without having to make incisions into the body, specifically the transanal cavity or colon, to access the affected area. This, thus, provides greater ease, and comfort for the patient, while also being a less expensive and less intrusive procedure than one involving surgical incisions to access the affected area. Furthermore, a surgical procedure involving surgical incisions is more demanding upon the body of a patient to recover from, as well as being a higher risk surgery for the patient, as incisions can increase the risk of infection and have other side effects. Thus, transanal endoscopic microsurgery (TEM) is a valuable surgical technique with a low complication rate for patients. In particular, TEM is an efficient method for patients with adenomatous rectal tumors and early rectal cancer.

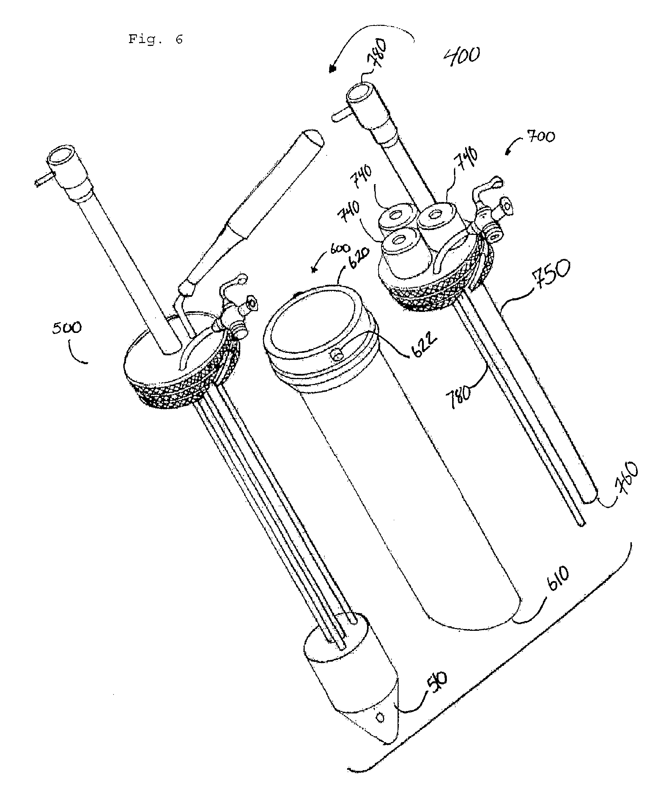

Typically, a rectoscope comprises a hollow cylindrical metal tube, having an outside diameter of 40 mm, and a length of 120 mm to 200 mm. For example, FIG. 6 shows a rectoscope instrument set 400 for performing transanal endoscopic surgical procedures. The rectoscope set 400 comprises an obturator 500, a rectoscope 600, and a boss element 700 for facilitating the insertion of additional surgical instruments into the rectoscope 600 while maintaining a pressure difference across the boss element 700 between the surgical cavity defined by the rectoscope 600, and the atmospheric air outside the rectoscope 600.

The rectoscope 600 has a distal end 610 and a proximal end 620. The distal end 610 is open. The proximal end 620 is open. The proximal end 620 is further provided with a locking element 622 for receiving either the obturator 500 or the boss element 700. When the proximal end 620 of the rectoscope 600 receives, for example, the boss element 700, the proximal 620 end of the rectoscope 600 is closed. It is preferred that the boss element 700 provides a fluid seal between rectoscope cavity and the atmospheric air during surgery while simultaneously providing instrument access across the fluid seal.

To insert a rectoscope 600 comfortably into the transanal cavity, surgeons typically use an obturator 500. An obturator 500, which is the central removable core of a rectoscope 600, allows for the easy insertion of the tip into the anus or another orifice.

During proctoscopy, the rectoscope 600 is lubricated and inserted into the rectum. The obturator 500 typically has a rounded distal end 510 which protrudes through the distal opening 610 of the rectoscope 600 during the insertion protocol. When inserted into the transanal cavity, the obturator 500 gradually expands the transanal cavity, thus allowing the surgeon to more easily access the cavity, and more easily insert the rectoscope 600.

The rectoscope 600 is inserted into the anal cavity until the distal open end 610 of the rectoscope 600 is proximate the surgical site. The obturator is then withdrawn from the rectoscope 600.

Next, the boss element 700 and its associated instruments are inserted through the open proximal end 620 of the rectoscope 600. The boss element 700 is then sealably secured to the proximal end 620 of the rectoscope. In reference to the boss element 700 shown in FIG. 6, the boss element 700 includes a linear optical element 750 inserted there through for use during the transanal endoscopic procedure. The boss element 700 further includes an insufflation channel 780 inserted there through for use during the transanal endoscopic procedure.

The insufflation channel 780 is used to expand the anal cavity during surgery with an insufflation gas. Insufflation involves an inert, nontoxic gas, such as carbon dioxide, being introduced into a body cavity, to expand the cavity. Insufflation is a common method to introduce compressed air into the transanal cavity, thus expanding the transanal cavity and reducing obstruction during investigative surgery and treatment.

In reference to FIG. 6, the boss element 700 further includes instrument ports 740 for receiving surgical instruments for use during the surgical procedure. The instrument ports 740 are designed to allow the shaft of a surgical instrument to pass through the port 740 while still maintaining a pressure difference across the boss element 700.

In reference to FIG. 6, the linear optical instrument 750 is inserted through the boss element 700. The linear optical element 750 is typically an endoscopic telescope or camera for use during a surgical procedure. The distal end 760 receives light at the surgical site and directs the light to the proximal end of the optical element 750 where the light provides an optical image of the affected site. The use of and design of optical elements is well known, and it should be understood that any known optical instrument, camera, or other visualization means may be used.

In some rectoscope sets 400, the optical instrument 750 is permanently integrated with the boss element 700. In other sets, the boss element 700 and the optical instrument 750 are separate and distinct pieces of surgical equipment. The optical instrument 750 has a distal end 760 and a proximal end 780. The optical instrument 750 disclosed in FIG. 6 extends along a single axis for its entire length. The distal end 760 of the optical instrument 750 extends at least to the distal end 610 of the rectoscope 600. The optical instrument 750 allows the surgeon to view the surgical site inside the anal cavity and perform the desired surgical procedure.

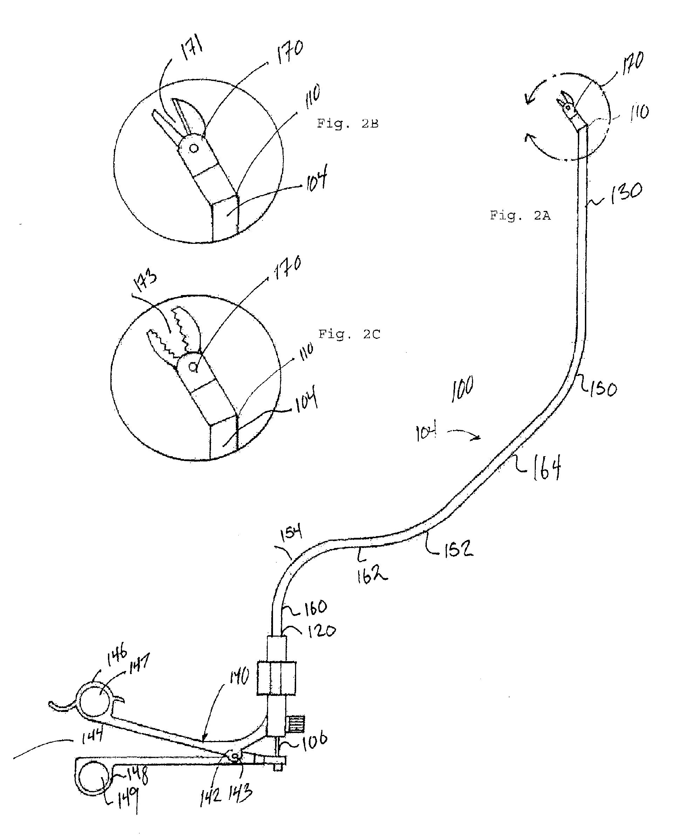

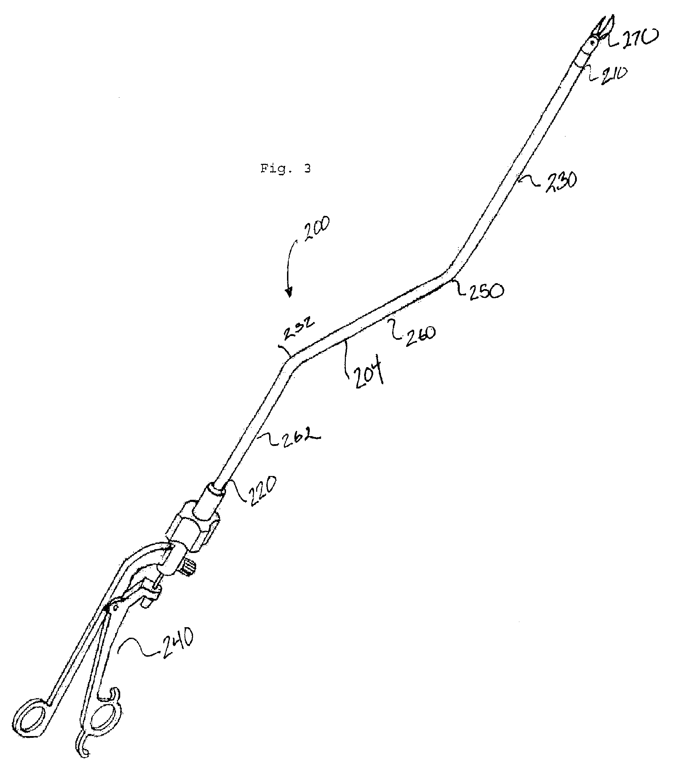

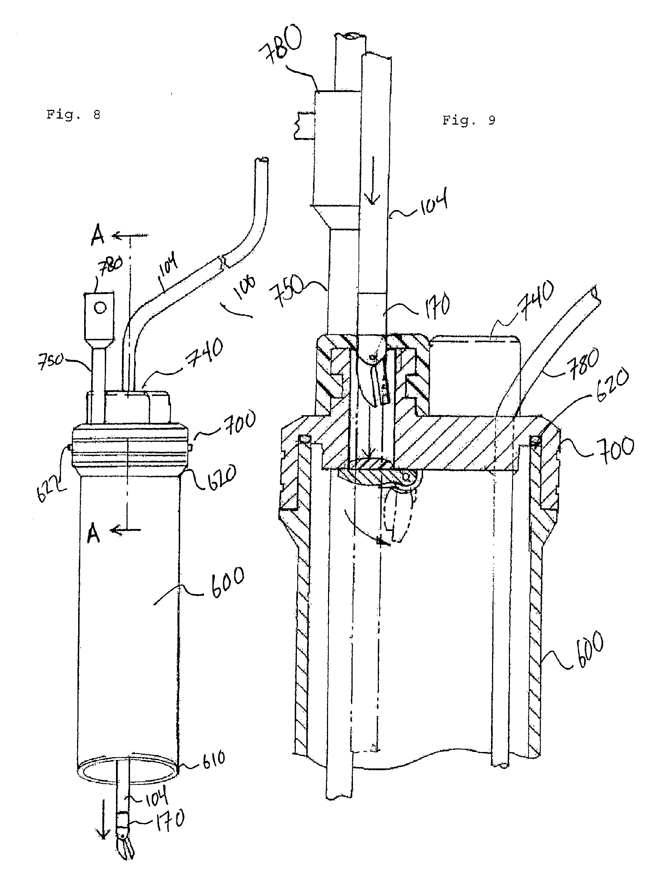

In reference to FIG. 8, a surgical instrument 100 is inserted into the instrument port 740. A portion of the instrument shaft 104 extends into the rectoscope 600. The distal end 110 of the instrument shaft 104 and the working part 170 attached to the distal end of the shaft extend past the distal open end 610 of the rectoscope 600. During a transanal surgical procedure a surgeon typically uses one or more surgical instruments 100 (shown in FIG. 2), 200 (shown in FIG.3) to operate on the surgical site.

A surgical instrument for performing transanal endoscopic surgery may comprise one of a member of one of the following subsets: graspers, scissors, gripper tools, biopsy tools, dissectors, and needle holders. Known surgical instruments for use in transanal endoscopy typically have an elongated shaft having a proximal end and a distal end. The known surgical instrument has a working part attached to the distal end of the shaft for operating on the affected area, and a handle portion attached to the proximal end of the shaft. The shaft has uniform cross section along its entire length. The shaft further extends along a single longitudinal axis

A disadvantage of known surgical instruments for use during endoscopic surgery, and more particularly transanal endoscopic surgery, is that it is difficult to access the proximal end of the instrument and actuate and manipulate the handle portion when the surgical instrument is inserted into a boss element of a rectoscope because the proximal end of the optical element interferences with the handle portion of the surgical instrument.

Typically a rectoscope, and as a result the boss element, has an outside diameter of approximately 40 mm. Therefore, there is limited space for the instruments and other optical elements being inserted through the instrument ports in the boss element to perform the surgical procedure. For example, when the linear surgical instrument and linear optical instrument are used together they are in close proximity. This is especially true because the optical instrument and the surgical instrument are substantially parallel as both the optical and surgical instrument must pass through the relatively narrow rectoscope cavity to access the surgical site. This configuration makes it very difficult to manipulate and actuate the handle portion of the surgical instrument. Likewise, it is difficult or near impossible to actuate and manipulate proximal end of the linear optical instrument.

Another disadvantage of the above described combination is that it is more difficult for the surgeon to generate the necessary force at the handle portion of the surgical instrument to actuate the working part of the surgical instrument due to the proximity of the handle portion of the surgical instrument to the proximal end of the linear optical element.

One possible solution to these problems is to use a non-linear optical element. For example the portion of the optical element extending outside the boss element during a surgery is directed away from the central axis of the rectoscope, thereby providing additional room to actuate the handle portions of the surgical instruments.

A disadvantage to this known solution is the increased cost of the unique optical element, as a non-linear optical element is very expensive. In contrast, a linear optical element is less expensive.

Another disadvantage of this known solution is that while positioning of the proximal end of the optical element may provide some additional room to actuate and manipulate the surgical instrument, it is difficult, if not impossible to perform an operation with two or three linear surgical instruments inserted through the instrument ports of the boss element.

Another disadvantage of this known solution is that it requires a specialized optical instrument customized for the procedure. Such an optical instrument is typically not available in the average operating room.

Another disadvantage of this known solution is that it does not allow for the use of linear optical elements that are typically available in the average operating room.

What is desired therefore is surgical instrument for use in combination with a linear optical element during a transanal endoscopic procedure, where the shape of the surgical instrument provides the necessary room to manipulate and actuate the proximal ends of one or more surgical instruments without interfering with the linear optical element.

What is further desired therefore is an endoscopic surgical instrument comprising an elongated shaft, said shaft having a proximal end and a distal end. The shaft having an insertion portion, the insertion portion extending along a longitudinal axis in a first plane. The surgical instrument further having a working part attached to the distal end of the shaft and a handle portion attached to the proximal end of the shaft. The handle portion being actuatable to actuate the working part via a force transmission member, and the proximal end of the shaft being laterally displaced relative to the longitudinal axis of the insertion portion.

### SUMMARY OF THE INVENTION

Accordingly, it is an object of the present invention to provide a surgical instrument and method that allows a surgeon to perform a transanal endoscopic surgical procedure or similar endoscopic procedure with a linear optical element.

It is another object of the present invention to provide a surgical instrument that allows a surgeon to perform a transanal endoscopic surgical procedure or similar endoscopic procedure with a linear optical element, wherein the surgical instrument provides sufficient clearance for the surgeon to actuate and manipulate the handle portion of the surgical instrument.

It is another object of the present invention to reduce the cost of transanal endoscopic surgery.

It is another object of the present invention to increase the acceptance of transanal endoscopic surgical techniques.

It is another object of the present invention to provide a surgical instrument that allows a surgeon to perform a transanal endoscopic surgical procedure or similar endoscopic procedure in which two or more surgical instruments are used to access the surgical site, wherein the shape of the surgical instruments provides sufficient clearance for the surgeon to actuate and manipulate the handle portion of each surgical instrument.

It is another object of the present invention to provide a surgical instrument having a shaft with a linear insertion portion extending along a longitudinal axis, wherein a proximal end of the shaft is laterally displaced from the longitudinal axis.

It is yet another object of the present invention to provide a surgical instrument having a shaft with a linear insertion portion extending along a longitudinal axis. The shaft further having one or more curved portions extending along a length of the shaft between the insertion portion and the proximal end, wherein the proximal end of the shaft is laterally displaced from the longitudinal axis of the insertion portion.

It is yet another object of the present invention to provide a surgical instrument having shaft with a linear insertion portion extending along a longitudinal axis. The shaft further having a first curved portion extending along a length of the shaft between the insertion portion and the proximal end, said first curve having a radius in a first plane, and a second curved portion extending along a length of the shaft between the first curved portion and the proximal end of the shaft, said second curved portion having a second radius in a second plane, wherein the proximal end of the shaft is laterally displaced from the longitudinal axis.

These and other objects of the present invention are achieved with an endoscopic surgical instrument comprising an elongated shaft, said shaft having a proximal end and a distal end. The shaft having an insertion portion, the insertion portion extending along a longitudinal axis in a first plane. The surgical instrument further having a working part attached to the distal end of the shaft and a handle portion attached to the proximal end of the shaft. The handle portion being actuatable to actuate the working part via a force transmission member, and the proximal end of the shaft being laterally displaced relative to the longitudinal axis of the insertion portion.

The invention and its particular features and advantages will become more apparent from the following detailed description considered with reference to the accompanying drawings.

### BRIEF DESCRIPTION OF THE DRAWINGS

Exemplary embodiments of the invention are explained in more detail in the description which follows and are represented in the drawings, in which:

FIG. 1 is a perspective view of a surgical instrument according to one embodiment of the present invention.

FIG. 2A is a front view of the surgical instrument shown in FIG. 1.

FIG. 2B is a front view of a scissors working part compatible with the surgical instrument shown in FIGS. 1 and 2A.

FIG. 2C is a front view of a gripper working part compatible with the surgical instrument shown in FIGS. 1 and 2A.

FIG. 3 is a perspective view of a surgical instrument according to a one embodiment of the present invention.

FIG. 4 is a front view of a surgical instrument according to one embodiment of the present invention.

FIG. 5 is a front view of a surgical instrument according to one embodiment of the present invention.

FIG. 6 is a perspective view of a rectocope instrument set for use during a transanal endoscopic surgical procedure.

FIG. 7 is a perspective view of a rectoscope and a distal end of a surgical instrument according to one embodiment of the present invention, wherein the broken lines illustrate the axial centerline of the instrument port for inserting the surgical instrument into the transanal cavity.

FIG. 8 is a front view of a rectoscope and a portion of a surgical instrument according to one embodiment of the present invention, wherein the insertion portion of the shaft of the surgical instrument is inserted through an instrument port of the rectoscope and the distal end of the surgical instrument extends through the open distal end of the rectoscope.

FIG. 9 is a cutaway view AA of the rectoscope and surgical instrument shown in FIG. 8.

FIG. 10 is a front view of a rectoscope and two surgical instruments according to an embodiment of the present invention, wherein the surgical instruments are inserted through instrument ports in the boss element and the working parts of the surgical instruments extend through the open distal end of the rectoscope.

### DETAILED DESCRIPTION OF THE INVENTION

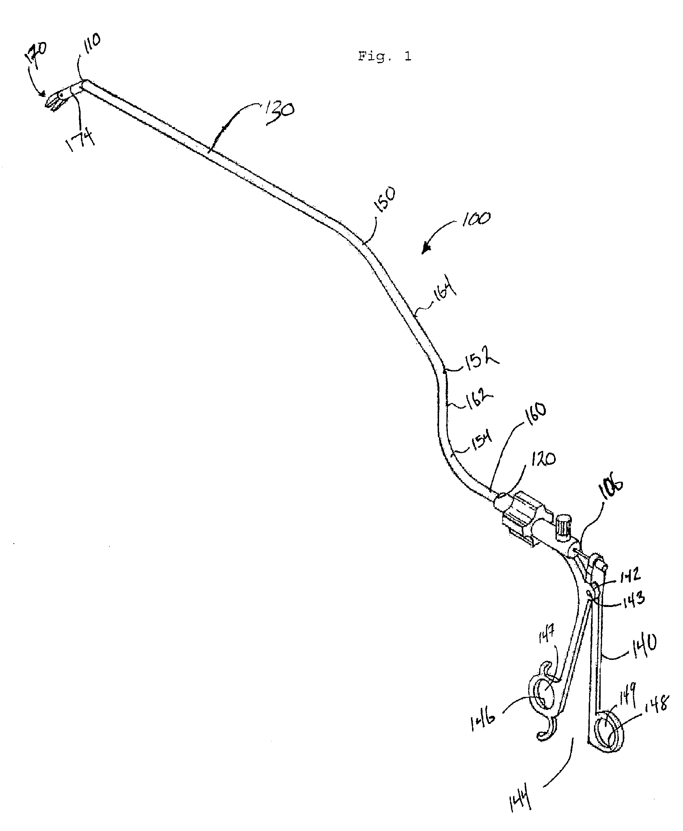

In reference to FIG. 1, a surgical instrument 100 in accordance with one embodiment of the present invention is shown. The surgical instrument 100 comprises an elongated rigid shaft 104 having a proximal end 120 and a distal end 110. The shaft 104 further has a linear insertion portion 130. The linear insertion portion 130 extends along a longitudinal axis in a first plane. The surgical instrument 100 further has a working part 170 attached to the distal end 110 of the shaft 104. In the embodiment disclosed in FIG. 1 the working part 170 is a scissors. The surgical instrument 100 further has a handle portion 140 attached to the proximal end 120 of the shaft 104. The working part 170 is connected to the handle portion 140 via a force transmission member 106. When an operator actuates the handle portion 140, it causes the force transmission member 106 to transmit a force to the working part 170 causing the working part 170, for example, to open and close.

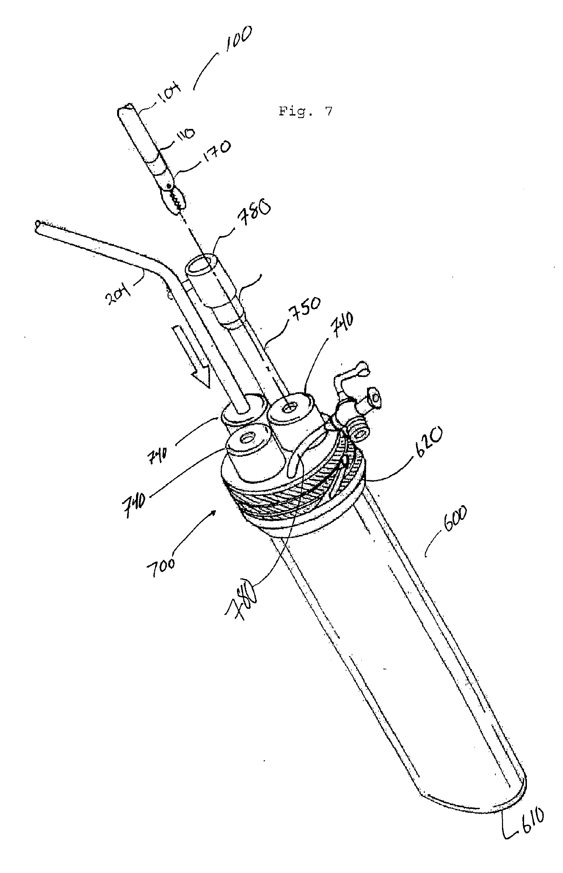

In further reference to the embodiment of the surgical instrument 100 shown in FIG. 1, the insertion portion 130 of the shaft 104 is typically inserted into a rectoscope 600 (shown in FIG. 6) during transanal endoscopic surgery. The insertion portion 130, along with the working part 170 attached to the distal end 110 of the shaft 104, is typically inserted through in instrument port 740 in a boss element 700 for use with a rectoscope 600, for example in reference to FIG. 7. Further referring to the embodiment disclosed in FIG. 1, the insertion portion 130 is about 200 mm along its longitudinal axis. This axial length provides the necessary length to insert the insertion portion 130 through the instrument port 740 (shown in FIG. 6) and provide access to the surgical site at the distal end 610 of the rectoscope 600. It should be understood that in some embodiments of the present invention the length of insertion portion 130 may vary, for example greater than 200 mm or, less than 50 mm. In some embodiments of the present invention the axial length of the insertion portion 130 is 100 mm.

Further referring to the surgical instrument 100 shown in FIG. 1, the insertion portion 130 has a uniform cross section along its length. The embodiment shown in FIG. 1 has a circular cross section having a diameter of 5 mm. The uniform diameter cross section allows the insertion portion 130 to create a fluid seal across the boss element 700 when the insertion portion 130 is inserted through an instrument port 740 in the boss element 700. The uniform cross section further ensures that the higher air pressure in the rectoscope cavity during surgery is not released when the insertion portion 130 is inserted through and moved around the instrument port 740. It should be understood that in some embodiments the insertion portion 130 does not have a uniform cross section or diameter along its length. For example, a constant cross section is not required for a surgical procedure performed at atmospheric pressure.

In further reference to the surgical instrument 100 shown in FIG. 1, the working part 170 is attached to the distal end 110 of the shaft 104. The working part 170 is typically a member of one of the following subsets: graspers, scissors, gripper tools, biopsy tools, dissectors, and needle holders. It should be understood that the working part 170, however, maybe of any type for use during a surgical procedure. The working part 170 disclosed in the embodiment shown in FIG. 1 is a scissors.

Further referring to the embodiment of the inventive surgical instrument 100 shown in FIG. 1, the working part 170 extends along a working part axis. In one embodiment of the present invention the working part axis is not parallel with the longitudinal axis of the insertion portion 130. For example the working part axis and the longitudinal axis of the insertion portion 130 may form an angle theta, wherein theta equals 35 degrees. It should be understood to a person having ordinary skill in the art that this angle may vary to accommodate certain types of procedures and different surgical sites. This configuration allows the surgeon to more easily access the surgical site located at the circumference of the rectoscope at the distal end of the rectoscope. In similar embodiments the shaft 104 comprises an extension portion 174 at its distal end 110 extending along an axis, and wherein the axis of said extension portion 174 is not parallel with said longitudinal axis of said insertion portion 130. The result is the same-easier access to the surgical site during transanal endoscopy.

The shaft 104 of the surgical instrument 100 disclosed in FIG. 1 includes three curved section 150, 152, and 154. The shaft 104 comprises a first curved portion 150 extending along a length of the shaft 104 between the insertion portion 130 and the proximal end 120 of the shaft 104. In the embodiment disclosed in FIG. 1, the first curved portion 150 is adjacent to the insertion portion 130. In the embodiment disclosed in FIG. 1, the radius of the first curve is 58 mm.

The shaft 104 of the surgical instrument 100 disclosed in FIG. 1, further includes a first linear portion 164 between the first curved portion 150 and the second curved portion 152. In the disclosed embodiment the first linear portion extends approximately 70 mm along the length of the shaft 104.

The shaft 104 of the surgical instrument 100 disclosed in FIG. 1 further includes a second curved portion 152 extending along a length of the shaft 104 between the first linear portion 164 and the proximal end 120 of the shaft 104. In the embodiment disclosed in FIG. 1, the radius of the second curve is 58 mm.

The shaft 104 of the surgical instrument 100 disclosed in FIG. 1, further includes a second linear portion 162 between the second curved portion 152 and the third curved portion 154. In the disclosed embodiment the second linear portion extends approximately 5 mm down the length of the shaft 104.

The shaft 104 of the surgical instrument 100 disclosed in FIG. 1 further includes a third curved portion 154 extending along a length of the shaft 104 between the second linear portion 162 and the proximal end 120 of the shaft. In the embodiment disclosed in FIG. 1, the radius of the third curve is 30 mm.

Finally, the shaft 104 of the surgical instrument 100 disclosed in FIG. 1, includes a third linear portion 160 between the third curved portion 154 and the proximal end 120 of the shaft 104. In the disclosed embodiment the third linear portion extends approximately 10 mm along the length of the shaft 104. The third linear portion extends along an axis that is parallel with the longitudinal axis of the insertion portion 130. The parallel insertion portion and distal third linear portion 160 allow for more precise manipulation of the surgical instrument 100.

The proximal end 120 of the shaft 104 of FIG. 1 is latterly displaced relative to the longitudinal axis of the insertion portion 130 as a result of the different curved and linear portions of the shaft between the insertion portion 130 and the proximal end 120 of the shaft 104. The lateral displacement of the proximal end 120 is measured along an imaginary line being substantially perpendicular to the longitudinal axis of the insertion portion 130. In the embodiment disclosed in FIG. 1, the lateral displacement is approximately 50 mm. The lateral displacement is achieved through the combination of linear and curved sections in the shaft 104 of the surgical instrument 100.

It should be understood that the lateral displacement of the proximal end 120 of the shaft 104 may be readily achieved through any number of different shaft 104 configurations readily discernable to any person having ordinary skill in the art. For example, in some embodiments the shaft 104 may include right angle portions, as opposed to the described curved portions. It should further be understood that the lateral displacement varies from different embodiments. For example, in some cases a lateral displacement of 10 mm is sufficient to overcome the problems with the prior art. In other case, the lateral displacement may be greater than 50 mm. The amount of lateral displacement depends on the diameter of the boss element 700, the type of surgical procedure, the preference of the surgeon, the type of patient, and the type of equipment used, among other factors.

Further referring to FIG. 1, the handle portion 140 is attached to the proximal end 120 of the shaft 104. The handle portion 140 is actuatable to actuate said working part 170 via a force transmission member 106. The handle portion 140 includes a scissors component 144 which is gripped by the surgeon and has front and rear scissors handles 146, 148 respectively. Scissors handles 146, 148 include finger loops 147, 149, and front scissors handle 146 includes a finger rest 141 (141 not in Fig 1 or Fig 2). Front scissors handle 146 includes a housing 142 to which scissors handle 148 is pivotably attached by a pivot pin 143. Thus, front scissors handle 146 is generally considered stationary, while rear scissors handle 148 is generally considered pivotable with respect thereto. The handle portion 140 typically extends along an axis substantially defined by the scissors component 144. In the embodiment shown in FIG. 1, the axis of the handle portion 140 is substantially perpendicular to the longitudinal axis of the insertion portion 130. This configuration directs the scissors component 144 of the handle portion 140, and handle portion 140 in general, away from the axial centerline of the rectoscope 600, thereby increasing the ease of access and use.

In reference to FIG. 2A, a front view of the surgical instrument 100 of FIG. 1 is shown. FIGS. 2B and 2C show an exploded view of the working part 170 and the distal end 110 of the shaft 104. In the embodiment shown in FIG. 2B the working part is a scissors 171. In the embodiment shown if FIG. 2C the working part is a gripper 173. The working part 170 is typically a member of one of the following subsets: graspers, scissors, gripper tools, biopsy tools, dissectors, and needle holders. It should be understood that the working part, however, may be of any type for use during an endoscopic procedure.

Another embodiment of a surgical instrument 200 in accordance with the present invention is shown in FIG. 3. The surgical instrument 200 comprises an elongated rigid shaft 204 having a proximal end 220 and a distal end 210. The shaft 204 further has a linear insertion portion 230. The linear insertion portion 230 extends along a longitudinal axis in a first plane for a distance of 200 mm. The surgical instrument 200 further has a working part 270 attached to the distal end 210 of the shaft 204. In the embodiment disclosed in FIG. 3 the working part 270 is a scissors. The surgical instrument 200 further has a handle portion 240 attached to the proximal end of the shaft 220. The handle portion 240 extends along an axis substantially parallel to the longitudinal axis of the insertion portion 230.

The shaft 204 of the surgical instrument 200 shown in FIG. 3 includes the insertion portion 230, two linear portions 260, 262, and two curved portions 250, 252 extending along the length of the shaft 204. The first curved portion 250 extends along a length of the shaft 204 between the insertion portion 230 and the first linear portion 260. The second curved portion 252 extends along a length of shaft 204 between the first linear portion 260 and the second linear portion 262. The second linear portion 262 is located at the proximal end 220 of the shaft 204.

In the embodiment disclosed in FIG. 3, the second linear portion 262 is substantially parallel with the longitudinal axis of the insertion portion 230. This combination achieves the necessary lateral displacement of the proximal end 220 of the shaft 204 so that the surgical instrument 200 does not interfere with a substantially linear optical instrument when both said surgical instrument and said optical instrument are simultaneously inserted into a rectoscope.

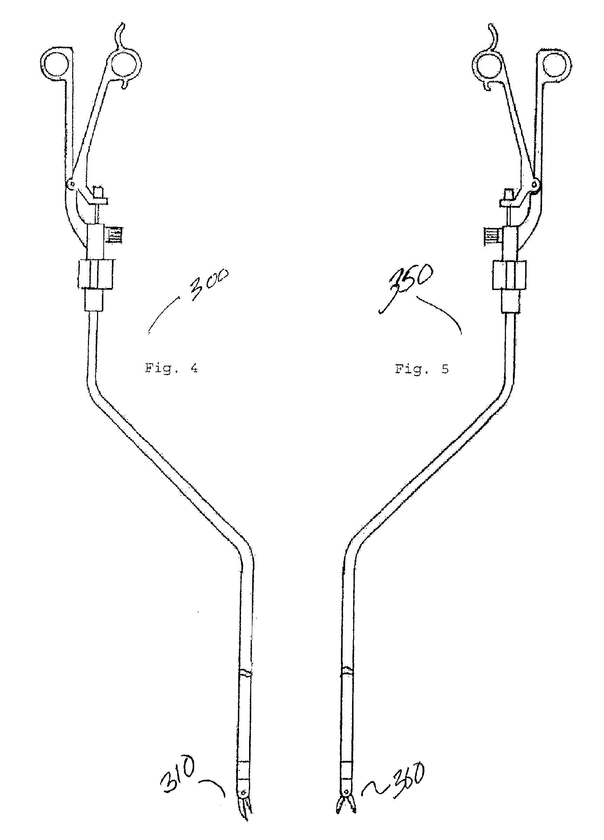

In reference to FIGS. 4 and 5, two surgical instruments in accordance with the present invention are shown on a single drawing page. FIG. 4 discloses a surgical instrument 300 having a scissors as a working part 310, and FIG. 5 discloses a surgical instrument 350 having a gripper as a working part 360. FIGS. 4 and 5 illustrate the advantages that the lateral displacement provides.

FIG. 7 shows a rectoscope 600 having a boss element 700 secured to its proximal end 620. FIG. 7 further discloses a portion of two surgical instruments 100, 200 in accordance with the present invention. Specially, FIG. 7 shows the shaft 204 of the first surgical instrument 200, wherein the distal end 210 of the shaft 204 and attached working part are inserted into an instrument port 740 in the boss element 700. FIG. 7 further discloses a linear optical element 750 inserted through the boss element 700. The insertion portion 230 and the linear optical element 750 are substantially parallel. However, the remaining portion of the shaft 204 laterally displaces the handle portion 240 (not shown) thereby providing additional room for the surgeon to actuate and manipulate the surgical instrument 200, and while at the same time providing additional room for the surgeon to actuate and manipulate the optical instrument 750.

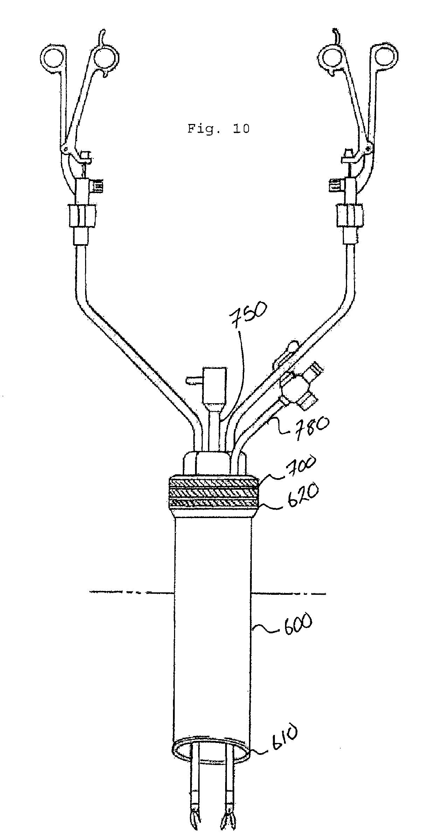

FIG. 10 shows two surgical instruments 800 and 900 in accordance with the present invention. FIG. 10 further shows a boss element 700 attached to the proximal end 620 of a rectoscope 600. The surgical instruments 800 and 900 are inserted into instrument ports 740 in the boss element 700. FIG. 10 further discloses a linear optical element 750 inserted through the boss element 700. The insertion portion 430 and the linear optical element 750 are substantially parallel. However, the remaining portion of the instrument shafts 804, 904 laterally displaces the handle portions 840, 940 thereby providing additional room for the surgeon to actuate and manipulate the surgical instruments, and also manipulate the linear optical element.

It should be understood that the foregoing is illustrative and not limiting, and that obvious modifications may be made by those skilled in the art without departing from the spirit of the invention. Accordingly, reference should be made primarily to the accompanying claims, rather than the foregoing specification, to determine the scope of the invention.

In order to summarize the foregoing, a surgical instrument adapted for use during transanal endoscopy is provided. The surgical instrument has a shaft having a proximal end and distal end. A working part, for example a scissors or gripper, is attached to the distal end of the shaft, and a handle portion is attached to proximal end of the shaft. The handle portion is actuatable to actuate said working part via a force transmission member. The shaft further includes a linear insertion portion having a longitudinal axis. The proximal end of the shaft is laterally displaced relative to the longitudinal axis of the insertion portion. This configuration increases the ability to manipulate and actuate the surgical instrument during a transanal endoscopic procedure or similar surgical procedure.

## Claims

1. An endoscopic surgical instrument, comprising:
an elongated shaft, said shaft having a proximal end and a distal end;
said shaft having a insertion portion, said insertion portion extending along a longitudinal axis in a first plane;
a working part attached to said distal end of said shaft;
a handle portion attached to said proximal end of said shaft, said handle portion being actuatable to actuate said working part via a force transmission member; and
wherein said proximal end of said shaft is laterally displaced relative to said longitudinal axis of said insertion portion.

2. The surgical instrument of claim 1, wherein said surgical instrument comprises a member of one of the following subsets: graspers, scissors, gripper tools, biopsy tools, dissectors, and needle holders.

3. The surgical instrument of claim 1 or 2, wherein said proximal end of said shaft is in said first plane.

4. The surgical instrument of anyone of claims 1 to 3, wherein said lateral displacement of said proximal end of said shaft is at least 30 mm, preferably at least 50 mm.

5. The surgical instrument of anyone of claims 1 to 4, wherein said insertion portion extends at least 100 mm, preferably at least 200 mm, along said longitudinal axis.

6. The surgical instrument of anyone of claims 1 to 5, wherein said shaft comprises one or more curved portions extending along a length of said shaft between said insertion portion and said proximal end.

7. The surgical instrument of anyone of claims 1 to 6, wherein said shaft comprises a linear portion at its proximal end, and said linear portion extending along an axis substantially parallel to said longitudinal axis of said shaft.

8. The surgical instrument of anyone of claims 1 to 7, wherein said handle portion extends along an axis substantially perpendicular to said longitudinal axis of said insertion portion.

9. The surgical instrument of anyone of claims 1 to 8, wherein said handle portion extends along an axis substantially parallel to said longitudinal axis of said insertion portion.

10. The surgical instrument of anyone of claims 1 to 9, wherein said insertion portion is adapted for insertion into an instrument port of a rectoscope.

11. The surgical instrument of anyone of claims 6 to 11, wherein said one or more curved portions of said shaft and said linear portion of said shaft are laterally displaced from said longitudinal axis of said insertion portion such that said surgical instrument does not interfere with a substantially linear optical instrument when both said surgical instrument and said optical instrument are simultaneously inserted into a rectoscope.

12. The surgical instrument of anyone of claims 1 to 11, wherein said shaft comprises an extension portion at its distal end extending along an axis, and
wherein said axis of said extension portion is not parallel with said longitudinal axis of said insertion portion.

13. The surgical instrument of anyone of claims 1 to 12, wherein said shaft comprises a first curved portion extending along a length of said shaft between said insertion portion and said linear portion, said first curved portion having a first radius.

14. The surgical instrument of claim 13, wherein said shaft comprises a second curved portion extending along a length of said shaft between said first curved portion and said linear portion of said shaft.

15. The surgical instrument of claim 14, wherein said shaft comprises a second linear portion extending along a length of said shaft between said first curved portion and said second curved portion.

16. The surgical instrument of anyone of claims 1 to 15, wherein said insertion portion has a uniform diameter along its longitudinal axis.

17. The surgical instrument of claim 16, wherein said uniform diameter is 5 mm.

18. The surgical instrument of anyone of claims 1 to 17, wherein
said shaft comprises a first curved portion extending along a length of said shaft in said first plane between said insertion portion and said linear portion, said first curved portion having a first radius;
said shaft comprises a second curved portion extending along a length of said shaft in said first plane between said first curved portion and said linear portion, said second curved portion having a second radius;
said shaft comprises a third curved portion extending along a length of said shaft in said first plane between said second curved portion and said linear portion, said third curved portion having a third radius;
wherein said first, second, and third radius are selected so that said instrument can be used in combination with a substantially linear optical instrument when said surgical instrument and said linear optical instrument are simultaneously inserted into a rectoscope.
